(19) Europäisches Patentamt
European Patent Office
Office européen des brevets

(11) EP 2 478 827 A1

(12) EUROPEAN PATENT APPLICATION

(43) Date of publication:
25.07.2012 Bulletin 2012/30

(51) Int Cl.:
A61B 1/07 (2006.01)   A61B 1/06 (2006.01)
A61B 1/04 (2006.01)   A61B 1/00 (2006.01)

(21) Application number: 11195902.9

(22) Date of filing: 28.12.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 19.01.2011 JP 2011008723

(71) Applicant: Fujifilm Corporation
Minato-ku
Tokyo (JP)

(72) Inventor: Terakawa, Yuki
Kanagawa (JP)

(74) Representative: Klunker . Schmitt-Nilson . Hirsch
Destouchesstraße 68
80796 München (DE)

(54) Endoscope system

(57) In an endoscope system having the function of capturing autofluorescence from a living body irradiated with excitation light and the function of capturing normal light observation images using white light as observation light, the normal light observation images are processed with a G fluorescence signal and an R fluorescence signal from the biological autofluorescence. The endoscope system produces bright images on which a lesion area can be easily identified in the endoscopic diagnosis using autofluorescence images.

FIG.3

**Description**

BACKGROUND OF THE INVENTION

The present invention relates to an endoscope system capable of making a diagnosis using biological autofluorescence. The invention more specifically relates to an endoscope system capable of obtaining a bright image in which a lesion area is easily identified by the biological autofluorescence.

Observation of the living body using an endoscope system is utilized in the medical field. So-called normal light observation (observation using white light as observation light) which involves irradiating an observation site of a living body with white light, receiving the light reflected on the living body with a CCD sensor or the like and converting the received light photoelectrically for photometry is commonly used in the endoscopic observation.

On the other hand, endoscope systems of a so-called autofluorescence observation type are also known recently which use a living tissue of a body emitting fluorescence by irradiation with ultraviolet light to blue light.

In autofluorescence observation, light in a predetermined wavelength range is irradiated on an observation site of a living body as excitation light and fluorescence generated in a living tissue irradiated with the excitation light is subjected to photometric measurement with a CCD sensor or the like. The intensity of the fluorescence generated in the living tissue irradiated with the excitation light is different between the normal area and the lesion area in the living body. Therefore, the normal area and the lesion area in the living body can be identified or discriminated from each other by making use of the autofluorescence from the living tissue.

For example, the systems described in JP 3810337 B and JP 2006-43289 A are known as the endoscope systems which perform the autofluorescence observation.

According to the system described in JP 3810337 B, excitation light is irradiated on an observation site and reflected light and autofluorescence from the observation site are separated into light in a first spectral range and light in a second spectral range, which are subjected to photometric measurement with a photodetector such as a CCD sensor. The first spectral range refers to one in which the autofluorescence intensity in the normal area is different from that in the lesion area. On the other hand, the second spectral range refers to one in which the autofluorescence intensity in the normal area is the same as that in the lesion area. In this system, the photometric results in the first spectral range are allocated to, for example, a G (green) channel and those in the second spectral range are allocated to, for example, an R (red) channel to generate a pseudo-color image, thereby performing the autofluorescence observation.

According to the endoscope system described in JP 2006-43289 A, illumination light in a predetermined wavelength range and excitation light capable of emitting fluorescence from a living tissue are successively irradiated on an observation site at predetermined intervals. The irradiation light is captured by a CCD sensor or the like to obtain a first image signal from the illumination light and a second image signal from the biological autofluorescence. The first and second image signals are stored, and one of the image signals is allocated to one or two of R, G and B (blue) channels for display and the other is allocated to the rest of the channels in synchronization with the above intervals. This system thus generates a pseudo-color image in the same manner as above to perform the autofluorescence observation.

SUMMARY OF THE INVENTIION

As described above, the intensity of the autofluorescence from the living tissue is different between the lesion area and the normal area. Therefore, images obtained in the autofluorescence observation can be used to identify the lesion area and the normal area. However, as also described in JP 3810337 B and JP 2006-43289 A, the conventional endoscope systems which perform the autofluorescence observation only display a pseudo-color image by receiving and photometrically measuring the autofluorescence from the living tissue or the reflected light from the living body and allocating the photometric results to the R, G and B channels corresponding to the display.

As is well known, the autofluorescence from the living tissue has a low intensity or a small amount of light emission. Therefore, in the pseudo-color image as described above, the lesion area and the normal area can often not be clearly identified.

In the conventional endoscope systems which perform the autofluorescence observation, image signals from the autofluorescence and those from the observation light in a predetermined wavelength range are used to generate a pseudo-color image. Therefore, the obtained image is wholly darker and lower in sharpness than the normal light image which uses white light as observation light.

An object of the present invention is to solve the foregoing prior art problems and to provide an endoscope system which performs the autofluorescence observation by photometrically measuring autofluorescence from a living tissue irradiated with excitation light and which is capable of clearly identifying a lesion area and a normal area with the autofluorescence from the living tissue and of obtaining a bright autofluorescence image.

In order to achieve the above object, the present invention provides an endoscope system comprising:

    a light source device comprising a light source for fluorescence observation which emits excitation light exciting a fluorescence component in a living body to cause the fluorescence component to emit fluorescence and a light source for normal light observation which emits white light;

an endoscope which receives the excitation light and the white light from the light source device, irradiates the excitation light and the white light into the living body and photoelectrically captures images in the living body; and

processing means for processing normal light observation images captured in the endoscope by irradiation with the white light, using a G fluorescence signal and an R fluorescence signal obtained by capturing in the endoscope the fluorescence emitted from the fluorescence component in the living body irradiated with the excitation light.

In the endoscope system of the invention, the light source for fluorescence observation preferably emits light at a wavelength of 370 to 470 nm as the excitation light.

The processing means preferably uses a ratio of the R fluorescence signal to the G fluorescence signal obtained by dividing the R fluorescence signal by the G fluorescence signal to process the normal light observation images.

The processing means preferably multiplies at least one of normal image signals including an R image signal, a G image signal and a B image signal by a correction factor including the ratio of the R fluorescence signal to the G fluorescence signal to process the normal light observation images.

The processing means preferably multiplies at least one of the normal image signals including the R image signal, the G image signal and the B image signal by the correction factor obtained by adding the ratio of the R fluorescence signal to the G fluorescence signal to 1 to process the normal light observation images.

Preferably, the processing means multiplies the ratio of the R fluorescence signal to the G fluorescence signal by a predetermined coefficient $\alpha$ and adds a resultant obtained by multiplication to 1 to generate the correction factor.

The processing means preferably multiplies at least one of the normal image signals including the R image signal, the G image signal and the B image signal by the correction factor obtained by subtracting the ratio of the R fluorescence signal to the G fluorescence signal from 1 to process the normal light observation images.

Preferably, the processing means multiplies the ratio of the R fluorescence signal to the G fluorescence signal by a predetermined coefficient $\alpha$ and subtracts a resultant obtained by multiplication from 1 to generate the correction factor.

The endoscope preferably comprises: a first solid-state image sensor for capturing the normal light observation images by irradiation with the white light; and a second solid-state image sensor for capturing the fluorescence emitted from the fluorescence component in the living body by irradiation with the excitation light.

The endoscope preferably uses one imaging lens to form the images in the first and second solid-state image sensors.

The second solid-state image sensor preferably captures the fluorescence emitted from the fluorescence component in the living body via a filter through which the excitation light does not pass but the fluorescence emitted from the fluorescence component in the living body passes.

The endoscope system of the invention configured as described above can enhance the lesion area by making use of the opposite properties of the autofluorescence in the green region and the autofluorescence in the red region in terms of the intensity in the lesion area and the normal area. In addition, the lesion area is enhanced using these properties on the basis of the normal light observation and therefore the autofluorescence image formed may have the same level of brightness as that of the normal light observation image.

Therefore, according to the invention, bright autofluorescence images can be used to correctly identify the lesion area and the normal area, thus making a proper diagnosis.

BRIEF DESCRIPTION OF THE DRAWINGS

FIG. 1 is a schematic perspective view showing an embodiment of an endoscope system of the invention.
FIG. 2 is a conceptual block diagram showing the configuration of the endoscope system shown in FIG. 1.
FIG. 3 is a conceptual block diagram showing a processor of the endoscope system shown in FIG. 1.
FIG. 4 shows spectra of autofluorescence emitted from a lesion area and a normal area of a living body.

DETAILED DESCRIPTION OF THE INVENTION

On the following pages, the endoscope system of the invention is described in detail with reference to the preferred embodiments illustrated in the accompanying drawings.

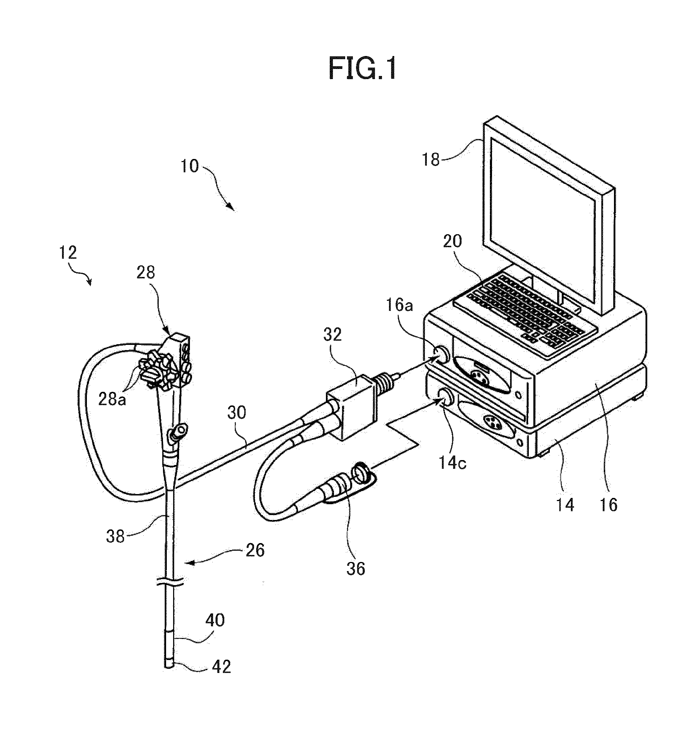

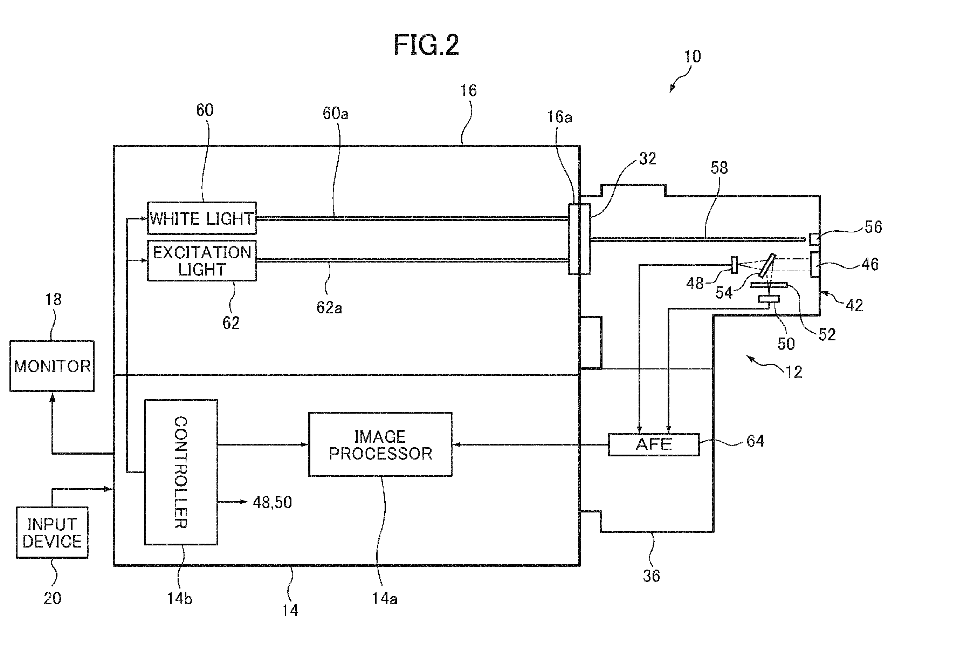

FIG. 1 is a schematic perspective view showing an embodiment of the endoscope system of the invention and FIG. 2 conceptually shows the configuration of the endoscope system shown in FIG. 1.

The illustrated endoscope system 10 includes an endoscope 12, a processing device 14 for processing an image captured by the endoscope 12 and a light source device 16 for supplying white light and excitation light for use in the observation and imaging using the endoscope 12.

The processing device 14 is connected to a monitor 18 for displaying an image captured by the endoscope and an input device 20 for inputting various instructions. The processing device 14 may further be connected to a printer (recording unit) for outputting an image captured by the endoscope as a hard copy.

The endoscope system 10 of the invention is a system capable of so-called normal light observation using white light as observation light and so-called autofluorescence observation (hereinafter referred to as "fluorescence observation") in which autofluorescence from a living tissue in an observation site exposed to excitation light is measured photometrically to form an image.

As shown in FIG. 2, the endoscope 12 is a so-called electronic endoscope which photoelectrically captures a biological image using an imaging device such as a CCD sensor 48. As in a common endoscope, the endoscope 12 includes an insertion section 26, an operating section 28, a universal cord 30, a connector 32 and a video connector 36.

During the observation (diagnosis), the endoscope 12 is used with the video connector 36 and the connector 32 connected to a connecting portion 14c of the processing device 14 and a connecting portion 16a of the light source device 16, respectively. As in a common endoscope, the connector 32 is connected to a suction means and an air supply means for the suction from and the air supply to the observation site, and a water supply means for the water injection on the observation site.

As in a common endoscope, the insertion section 26 of the endoscope 12 includes a long flexible portion 38 on the proximal side, a distal scope portion (endoscope distal portion) 42 provided with the CCD sensor 48 and the like, and a bending portion (angle portion) 40 located between the flexible portion 38 and the scope portion 42. The operating section 28 of the endoscope 12 includes manipulation knobs 28a for bending the bending portion 40 and an imaging button for capturing still images.

As schematically shown in FIG. 2, the scope portion 42 is provided with an imaging lens 46, the CCD sensor 48, a high-sensitivity CCD sensor 50, an excitation light cut filter 52, a half mirror 54, an illumination lens 56 and an optical fiber 58. The scope portion 42 also includes a cover glass (not shown) for protecting the lenses and the like.

Although not shown, the endoscope 12 is also provided with a forceps channel and a forceps port for inserting various treatment tools such as a forceps, and air supply/water supply channels and air supply/water supply ports for use in suction, air supply and water supply.

The forceps channel extends through the bending portion 40 and the flexible portion 38 to communicate with a forceps insertion port provided in the operating section 28. The air supply/water supply channels extend through the bending portion 40, the flexible portion 38, the operating section 28 and the universal cord 30 to communicate with connecting portions with the suction means, the air supply means and the water supply means in the connector 32.

The optical fiber 58 extends through the bending portion 40, the flexible portion 38, the operating section 28 and the universal cord 30 and terminated by the connector 32 which is connected to the light source device 16.

White light and excitation light emitted from the light source device 16 to be described later enters the optical fiber 58 through the connector 32 and propagates through the optical fiber 58. In the scope portion 42, the light enters the illumination lens 56 from the distal end of the optical fiber 58 and passes through the illumination lens 56 to be irradiated on an observation site.

Reflected light from the observation site of the living body irradiated with white light is imaged by the CCD sensor 48. Autofluorescence (hereinafter referred to as "fluorescence") from a living tissue in the observation site exposed to excitation light is imaged by the high-sensitivity CCD sensor 50.

The CCD sensor 48 is a common color CCD sensor which measures R (red) light, G (green) light and B (blue) light photometrically to form (capture) color images and is used in a common endoscope or digital camera. Therefore, the CCD sensor 48 outputs signals of an R image, a G image and a B image.

On the other hand, the high-sensitivity CCD sensor 50 (hereinafter referred to as "high-sensitivity sensor 50") is the one which is used to measure R light and G light photometrically and which can also detect weak incident light with a small quantity of light to output signals. Therefore, the high-sensitivity sensor 50 outputs signals of an R image and a G image.

The scope portion 42 including the CCD sensor 48 and high-sensitivity sensor 50 as described above will be described later in detail.

In the practice of the invention, the imaging device is not limited to the CCD sensor 48 and various known imaging devices such as a CMOS image sensor may be used.

A high-sensitivity CCD sensor which measures R light, G light and B light photometrically as in a common color CCD sensor may be used as the high-sensitivity sensor 50 so that fluorescence observation to be described later is performed only using output signals from R and G pixels.

Output signals from the CCD sensor 48 and the high-sensitivity sensor 50 are sent on signal lines from the scope portion 42 to the video connector 36 through the bending portion 40, the flexible portion 38, the operating section 28, the universal cord 30 and the connector 32.

In the illustrated embodiment, an AFE (Analog Front End) board 64 is disposed in the video connector 36.

The AFE board 64 includes, for example, a correlated double sampling circuit, an amplifier (automatic gain control circuit) and an A/D converter. In the AFE board 64, the output signals from the CCD sensor 48 and the high-sensitivity sensor 50 are subjected to noise removal by correlated double sampling and amplification in the amplifier. The amplified signals are further converted in the A/D converter from analog form to digital form to obtain digital image signals. The digital image signals are then outputted to the processing device 14 (more specifically to a DSP 72 to be described later).

In the endoscope system of the invention, these processing steps may be performed in the connector 32 or the processing device 14 instead of the video connector 36.

As described above, the connector 32 of the endoscope 12 in the endoscope system 10 is connected to the connecting portion 16a of the light source device 16.

The light source device 16 supplies to the endoscope 12 white light for the normal light observation in the living body and excitation light for emitting fluorescence from the living tissue. As described above, the white light and excitation light supplied from the light source device 16 toward the endoscope 12 enters the optical fiber 58 through the connector 32 and propagates therethrough to be irradiated on the observation site through the scope portion 42 at the distal end of the insertion section 26.

As schematically shown in FIG. 2, the light source device 16 includes a white light source 60 for emitting white light for use in the normal light observation, an excitation light source 62 for emitting excitation light for use in the fluorescence observation, and optical fibers 60a and 62a.

The white light source 60 emits white light which is observation light for use in the so-called normal light observation in the endoscope.

In the illustrated endoscope system 10, the normal light observation using white light as observation light can be performed by capturing an image only using the white light source 60 as the light source and the CCD sensor 48 (color CCD sensor) as the imaging means.

The white light source 60 is not particularly limited and various light sources capable of emitting white light and used in endoscope systems, as exemplified by a xenon lamp and a natural light LED may be employed.

A light source (light source device) using given phosphors which is illustrated in commonly assigned JP 2009-56248 A is also advantageously used for the white light source 60. Light in a first wavelength range emitted from the light source excites the phosphors to generate light in a second wavelength range and the light in the first wavelength range is combined with the light in the second wavelength range to generate white illumination light.

An example of the white light source using the phosphors as described above is the one which includes a laser light source emitting B light at a central wavelength of 445 nm and a plurality of kinds of phosphors (e.g., YAG phosphor and BAM ($BaMgAl_{10}O_{17}$) phosphor) which absorb part of the B light, excite and emit green to yellow light.

The green to yellow light emitted from the phosphors excited by the B light is combined with part of the B light which was not absorbed in the phosphors but passed therethrough to form pseudo white light.

Such a white light source using the phosphors may be combined with a laser light source emitting a blue-violet laser beam at a central wavelength of 405 nm, which also enables so-called special light observation (narrowband light observation) using narrowband light as observation light.

The blue-violet laser beam at the central wavelength of 405 nm emitted from the laser light source is hardly absorbed in the phosphors and passes therethrough. Therefore, by using the laser light source of the blue-violet laser beam in combination, the blue-violet narrowband light is added to the white light as observation light to enable special light imaging with narrowband light to be performed. In addition, the normal light observation and special light observation can be performed substantially at the same time by turning on and off the laser light source of the blue-violet laser beam at predetermined intervals (or high power lighting and low power lighting are alternately repeated) with the laser light source of the B light kept turned on.

On the other hand, the excitation light source 62 is a light source emitting excitation light which excites living tissues of the body such as porphyrin, NADH (reduced nicotinamide adenine dinucleotide), NADPH (reduced nicotinamide adenine dinucleotide phosphate) and FAD (flavin adenine dinucleotide) to emit (auto)fluorescence.

The excitation light is not particularly limited and light at any wavelength capable of exciting a living tissue to cause fluorescence therein can be all used.

In particular, light at a wavelength of 370 to 470 nm (light having a peak (highest intensity) within this wavelength range) is advantageously used as the excitation light from the viewpoint that the opposite properties of R fluorescence and G fluorescence from the living tissue in terms of the emission intensity which will be described later can be advantageously exhibited. Light at a wavelength of 400 to 450 nm can be more advantageously used because the opposite properties are exhibited and the biological safety can be more reliably ensured.

Various light sources capable of emitting, at a sufficient intensity, excitation light causing fluorescence in living tissues can be all used for the excitation light source 62.

For example, various laser light sources such as a laser diode (LD) having a peak within the foregoing wavelength range are preferably used for the excitation light source 62. A light source such as the one described in JP 2006-43289 A which uses a white light source such as a xenon lamp and a filter passing light in a predetermined wavelength range corresponding to the excitation light can also be used for the excitation light source 62.

The white light emitted from the white light source 60 (observation light used in the normal light observation) is propagated through the optical fiber 60a, is supplied through the connecting portion 16a to the connector 32 of the endoscope 12 and then supplied to the optical fiber 58 from the connector 32. On the other hand, the excitation light emitted from the excitation light source 62 is propagated through the optical fiber 62a, is likewise supplied through the connection portion 16a to the connector 32 of the endoscope 12 and then supplied to the optical fiber 58 from the connector 32.

The white light and excitation light having been supplied to the optical fiber 58 are propagated through the optical fiber 58, exit from the distal end of the optical fiber 58 on the side of the scope portion 42 and pass through the illumination lens 56 to be irradiated on the observation site in the living body.

Reflected light from the observation site of the body irradiated with the white light and fluorescence emitted from the living tissue in the observation site irradiated with the excitation light are captured and imaged via the imaging lens 46 and the half mirror 54 by the CCD sensor 48 and the high-sensitivity sensor 50, respectively.

More specifically, the half mirror 54 is disposed on the optical axis of the imaging lens 46 in the scope portion 42. Light which passed through the half mirror 54 is incident on the CCD sensor 48 to form an image whereas light which was reflected on the half mirror 54 is incident on the excitation light cut filter 52, through which light at wavelengths outside the wavelength band of the excitation light passes to be incident on the high-sensitivity sensor 50 to form an image therein.

Various filters which block the excitation light and pass light on the longer wavelength side than the wavelength band of the excitation light (wavelength band including at least an R light region) can be used for the excitation light cut filter 52.

An exemplary filter that may be used includes one which blocks light at wavelengths equal to or shorter than the maximum wavelength of the excitation light and passes light on the longer wavelength side than the maximum wavelength of the excitation light. For example, when the excitation light source 62 is a laser light source such as an LD, a filter which blocks light at wavelengths equal to or shorter than the central wavelength + 20 nm and preferably the central wavelength + 10 nm, and passes light at wavelengths on the longer wavelength side may be used. More specifically, when the excitation light source 62 is a laser light source having a central wavelength of 400 nm, the excitation light cut filter 52 which blocks light at wavelengths equal to or shorter than 420 nm and preferably equal to or shorter than 410 nm, and passes light at wavelengths on the longer wavelength side may be used.

When the excitation light is produced by a band-limiting filter such as the one described in JP 2006-43289 A, various known excitation light cut filters including the one described therein can be used.

When the normal light observation is performed in the endoscope system 10, the white light is continuously emitted only from the white light source 60 of the light source device 16 to image only in the CCD sensor 48 thereby capturing normal light observation images as in the normal light observation using a common endoscope system.

On the other hand, when the fluorescence observation is performed, the white light source 60 and the excitation light source 62 of the light source device 16 are alternately turned on (driven) at predetermined intervals under the control of a controller 14b of the processing device 14 to be described later. In other words, when the fluorescence observation is performed in the endoscope system 10, the white light as the observation light for the normal light observation and the excitation light for the fluorescence observation are alternately irradiated on the observation site at predetermined intervals.

The intervals at which the white light source 60 and the excitation light source 62 are alternately turned on may be appropriately set according to the sensor sensitivity and the site to be imaged.

When the fluorescence observation is performed, the endoscope 12 which is under the control of the controller 14b uses the CCD sensor 48 to perform imaging (sampling) of the white light emitted from the white light source 60 and uses the high-sensitivity sensor 50 to perform imaging of the excitation light emitted from the excitation light source 62.

In other words, when the fluorescence observation is performed in the endoscope system 10, formation of the normal light images (R image, G image and B image) in the CCD sensor 48 and formation of the fluorescence images (R image and G image) in the high-sensitivity sensor 50 are alternately performed in response to (in synchronization with) the alternate lighting of the white light source 60 and the excitation light source 62 at the predetermined intervals.

The invention uses the half mirror 54 to change the optical path to supply light to one of the CCD sensor 48 and the high-sensitivity sensor 50 but the invention is not limited to this configuration. For example, a total reflection mirror may be inserted into or retracted from the optical path so that the subsequent optical path is directed to the CCD sensor 48 or the high-sensitivity sensor 50.

As shown, the endoscope used in the endoscope system 10 of the invention is of a so-called one-lens two-sensor configuration in which two imaging devices for use in the normal light observation and fluorescence observation are provided for one imaging lens 46, and the CCD sensor 48 and the high-sensitivity sensor 50 are selectively used by changing the optical path. However, the invention is not limited to this. In other words, various configurations are possible in the endoscope system 10 of the invention as long as the same observation site can be alternately imaged by the normal light observation using the white light and the fluorescence observation with the excitation light cut off.

For example, a two-lens two-sensor configuration may be used in which an imaging device for use in the normal light observation and an imaging device for use in the fluorescence observation each have the corresponding imaging lens.

Alternatively, one-lens one-sensor configuration is also possible if the imaging device has sufficient sensitivity. This configuration is exemplified by the case in which, for example, a filter turret having a hole and an excitation light cut filter is provided between the imaging lens and the imaging device to alternately perform the normal light observation and the fluorescence observation.

As described above, output signals from the CCD sensor 48 and the high-sensitivity sensor 50 are sent to the video connector 36, subjected to processing such as A/D conversion in the AFE 64 and supplied to the processing device 14 (more specifically a processor 14a thereof) as a digital image (digital image signals (image data/image information)).

The processing device 14 subjects the normal light observation images and fluorescence images (hereinafter also collectively referred to simply as "images" when it is not necessary to distinguish them from each other) supplied (outputted) from the endoscope 12 to predetermined processing so that the monitor 18 displays them as the images captured by the endoscope 12 and also controls the endoscope system 10.

In the illustrated embodiment, the processing device 14 includes the image processor 14a and the controller 14b for controlling the whole of the endoscope system 10 including the processing device 14.

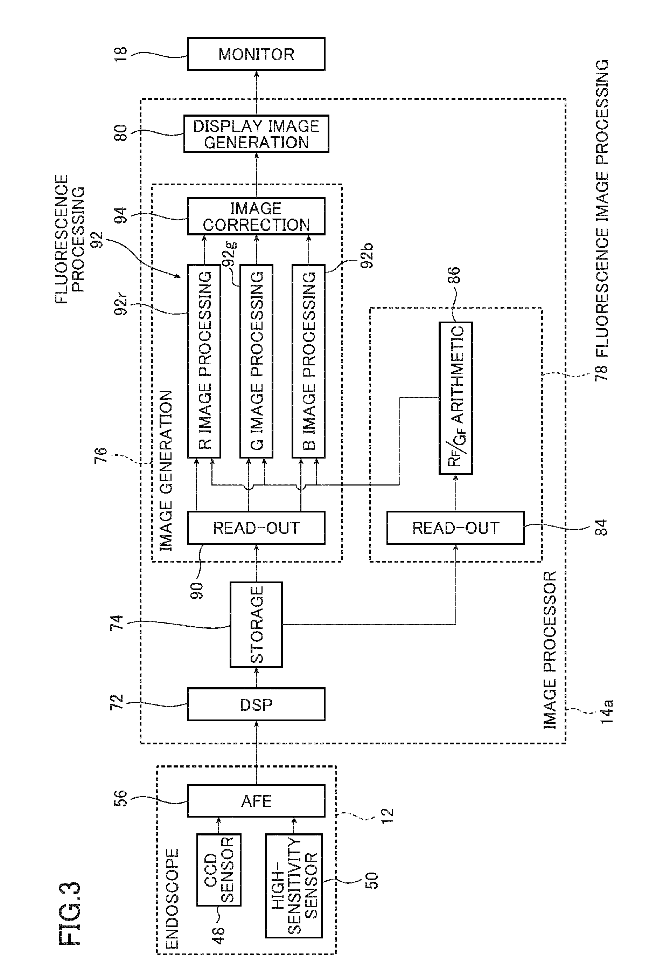

FIG. 3 is a conceptual block diagram showing the image processor 14a of the processing device 14.

As shown in FIG. 3, the image processor 14a includes the DSP 72, a storage section 74, an image generating section 76, a fluorescence image processing section 78 and a display image generating section 80.

In the processing device 14, images from the endoscope 12 are supplied to the DSP 72.

The DSP 72 is a known type of DSP (Digital Signal Processor), where the supplied images are subjected to predetermined processing steps such as gamma correction and color correction. The processed images are then stored in a predetermined region of the storage section (memory) 74.

Upon the storage of the images in the storage section 74, the image generating section 76 or the fluorescence image processing section 78 reads out images and performs predetermined processing on the read-out images.

The fluorescence image processing section 78 includes a read-out portion 84 and an $R_F/G_F$ arithmetic portion 86. The fluorescence image processing section 78 only functions when the fluorescence observation is performed.

Once the storage section 74 stores the images captured by the high-sensitivity sensor 50, the read-out portion 84 of the fluorescence image processing section 78 which is under the control of the controller 14b reads out the images and supplies them to the $R_F/G_F$ arithmetic portion 86. As described above, the high-sensitivity sensor 50 measures R light and G light photometrically to form an image of (auto)fluorescence from the living tissue exposed to the excitation light. Therefore, an R fluorescence image $R_F$ and a G fluorescence image $G_F$ are read out by the read-out portion 84 and supplied to the $R_F/G_F$ arithmetic portion 86.

The $R_F/G_F$ arithmetic portion 86 uses the supplied fluorescence images $R_F$ and $G_F$ to calculate the ratio $R_F/G_F$ between the fluorescence image $R_F$ and the fluorescence image $G_F$ and supply the calculated ratio to the fluorescence processing portion 92 of the image generating section 76 to be described later.

On the other hand, the image generating section 76 includes a read-out portion 90, the fluorescence processing portion 92 and an image correcting portion 94.

Once the storage section 74 stores the images captured by the CCD sensor 48, the read-out portion 90 of the image generating section 76 which is under the control of the controller 14b reads out the images. As described above, the CCD sensor 48 is a common color CCD sensor and captures normal light images. Therefore, the read-out portion 84 reads out an R normal image $R_N$, a G normal image $G_N$ and a B normal image $B_N$.

The fluorescence processing portion 92 only functions when the fluorescence imaging is performed. Therefore, when the normal light observation is performed, the normal images $R_N$, $G_N$ and $B_N$ read out by the read-out portion 90 pass through the fluorescence processing portion 92 to be directly supplied to the image correcting portion 94 as an R image $R_D$, a G image $G_D$ and a B image $B_D$ without being processed in the fluorescence processing portion 92.

The image correcting portion 94 subjects the R, G and B images to processing with a 3x3 matrix, gradation conversion, processing with a three-dimensional LUT or other color conversion processing; color enhancement for giving a color difference between a blood vessel and a mucous membrane on the screen by enhancing in a direction in which the color difference between the blood vessel and the mucous membrane is to be more accentuated than the average colors of the image so that the blood vessel can be more easily seen; image structure enhancement such as sharpening and edge enhancement, and other image correction steps and supplies the corrected image to the display image generating section 80 as the images for display.

In contrast, when the fluorescence observation is performed, the normal images $R_N$, $G_N$ and $B_N$ read out by the read-out portion 90 are supplied to the image correcting portion 94 as an R image $R_D$, a G image $G_D$ and a B image $B_D$ after being processed in the fluorescence processing portion 92. The image correcting portion 94 performs the same image correction as above and sends the corrected images to the display image generating section 80 as the images for display.

The fluorescence processing portion 92 includes an R image processing portion 92r, a G image processing portion 92g and a B image processing portion 92b. The image processing portions process the normal images (respective pixels thereof) using the ratio $R_F/G_F$ calculated by the $R_F/G_F$ arithmetic portion 86 ($R_F/G_F$ for the respective pixels of the normal images). In the fluorescence processing portion 92, the R image processing portion 92r processes the normal image $R_N$ to generate the R image $R_D$, the G image processing portion 92g processes the normal image $G_N$ to generate the G image $G_D$ and the B image processing portion 92b processes the normal image $B_N$ to generate the B image $B_D$.

More specifically, in the illustrated case, the image processing portions of the fluorescence processing portion 92 process the normal images $R_N$, $G_N$ and $B_N$ with $R_F/G_F$ to generate the images $R_D$, $G_D$ and $B_D$, respectively.

$$R_D = R_N \times \left| 1 + \alpha \times \frac{R_F}{G_F} \right|$$

$$G_D = G_N \times \left| 1 - \alpha \times \frac{R_F}{G_F} \right|$$

$$B_D = B_N \times \left| 1 - \alpha \times \frac{R_F}{G_F} \right|$$

In the above formula, $\alpha$ is a preferably used coefficient which is appropriately set so that $R_F/G_F$ in the lesion area may approach 1. $\alpha$ is more preferably a coefficient appropriately set so that $R_F/G_F$ in the lesion area may approach 1 and $R_F/G_F$ in the normal area may approach 0.

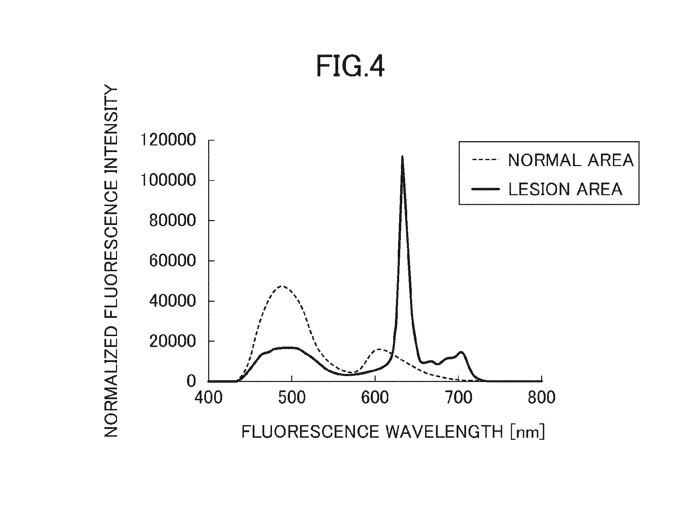

FIG. 4 shows spectra of (auto)fluorescence from a living tissue when a living body (human colonic mucosa) is irradiated with light at a central wavelength of 380 nm emitted from the laser light source as excitation light.

In FIG. 4, the horizontal axis shows the fluorescence wavelength and the vertical axis shows the fluorescence intensity. In FIG. 4, the solid line shows the fluorescence from the lesion area and the broken line shows the fluorescence from the normal area.

In the fluorescence generated by exposure to B light or blue-violet light as excitation light, it is considered that the fluorescence image $R_F$ is mainly formed by R fluorescence due to porphyrin and the fluorescence image $G_F$ is mainly formed by G fluorescence due to NADH and NADPH.

As for the R fluorescence making up the fluorescence image $R_F$, as is seen from, for example, the fluorescence property at around 630 nm, the normal area does not show very strong fluorescence whereas the lesion area shows very strong fluorescence. On the other hand, as for the G fluorescence making up the fluorescence image $G_F$, as is seen from, for example, the fluorescence property at around 470 nm to 530 nm, the normal area shows strong fluorescence whereas the lesion area does not show very strong fluorescence.

As is seen from above, R fluorescence and G fluorescence generated from the living tissue exposed to B light as excitation light have opposite properties in terms of the intensity in the normal area and the lesion area. In other words, the fluorescence image $R_F$ and the fluorescence image $G_F$ obtained by exposure to B light as excitation light have opposite properties in terms of the intensity in the normal area and the lesion area.

The lesion area can be enhanced by making use of the opposite properties of the fluorescence image $R_F$ and the fluorescence image $G_F$ in the lesion area and the normal area.

More specifically, the lesion area has a higher intensity in the fluorescence image $R_F$ and a lower intensity in the fluorescence image $G_F$ and the ratio $R_F/G_F$ is therefore increased. On the other hand, the normal area has a lower intensity in the fluorescence image $R_F$ and a higher intensity in the fluorescence image $G_F$ and the ratio $R_F/G_F$ is therefore decreased. Accordingly, this relation is used to process a normal image of an appropriately selected color by, for example, multiplication, addition or subtraction of $R_F/G_F$ to enhance the lesion area or suppress the normal area in the normal image, thus enabling an image with enhanced lesion area to be obtained.

In the illustrated case, use is preferably made of the coefficient $\alpha$ which is set so that $R_F/G_F$ is closer to 1 in the lesion area having a larger $R_F/G_F$ and is closer to 0 in the normal area having a smaller $R_F/G_F$.

In the R image, $\alpha \times R_F/G_F$ is added to 1 and multiplied by the normal image $R_N$ to calculate the image $R_D$. On the other hand, in the G and B images, $\alpha \times R_F/G_F$ is subtracted from 1 and multiplied by the normal images $G_N$ and $B_N$ to calculate the images $G_D$ and $B_D$, respectively.

In each of the normal images processed in the fluorescence processing portion 92, $R_F/G_F$ is or is close to 0 in the normal area and the normal images including R, G and B images are multiplied by 1 or a value close to 1. Therefore, the normal area has no large image changes between the normal images and the images $R_D$, $G_D$ and $B_D$, respectively.

In contrast, $R_F/G_F$ is or is close to 1 in the lesion area. Therefore, in each of the normal images processed in the fluorescence processing portion 92, the normal image $R_N$ is multiplied by 2 or a value close to 2 in the lesion area and the image $R_D$ is enhanced twice as large as that of the normal image $R_N$. In the lesion area, the normal images $G_N$ and $B_N$ are both multiplied by 0 or a value closer to 0 and the images $G_D$ and $B_D$ both have values close to 0.

In other words, in this example, the lesion area of the obtained image is enhanced with red with respect to the normal area.

As is clear from the above description, by making use of the opposite properties of the fluorescence images $R_F$ and $G_F$ in the normal area and lesion area (i.e., the opposite properties of the R fluorescence and the G fluorescence), the lesion area can be highlighted with a predetermined color in the fluorescence image. In addition, the normal light observation image used as the base is highlighted in the fluorescent image and therefore the displayed fluorescence image has the same level of brightness as that of the common normal light observation image.

Therefore, according to the endoscope system of the invention, bright fluorescence images with enhanced lesion area can be used to correctly identify the lesion area and the normal area.

As described above, the coefficient $\alpha$ is preferably used in the processing of the normal images with $R_F/G_F$ in the fluorescence processing portion 92 and is appropriately set so that $R_F/G_F$ may approach 1 in the lesion area. The coefficient $\alpha$ is more preferably a coefficient appropriately set so that $R_F/G_F$ in the lesion area may approach 1 and $R_F/G_F$ in the normal area may approach 0.

Use of the coefficient $\alpha$ as described above enables the lesion area to be suitably enhanced in a well-balanced manner while preventing the image of each color from being unnecessarily increased or decreased.

The coefficient $\alpha$ may be appropriately set according to the device characteristics of the endoscope 12 and the light source device 14 so that $R_F/G_F$ may approach or be 1 in the lesion area. More specifically, the coefficient $\alpha$ may be appropriately set according to the device characteristics such as the spectral sensitivity characteristics of the high-sensitivity sensor 50 (band characteristics of the filter and the spectral sensitivity of the device), the wavelength of the excitation light emitted from the light source device 16 (spectral characteristics), and the spectral characteristics of the excitation light cut filter 52 (filter characteristics) so that $R_F/G_F$ may approach 1 in the lesion area. Preferably, the coefficient $\alpha$ is appropriately set according to the device characteristics so that $R_F/G_F$ may approach 1 in the lesion area and approach or be 0 in the normal area.

In the above example, $\alpha \times R_F/G_F$ is added to 1 in the R image to calculate the image $R_D$ (this operation is hereinafter referred to simply as "addition"), and $\alpha \times R_F/G_F$ is subtracted from 1 in the G and B images to calculate the images $G_D$ and $B_D$ (this operation is hereinafter referred to simply as "subtraction") to enhance the lesion area with red.

However, the invention is not limited to this and various combinations of addition and subtraction processing of the normal images can be used in the fluorescence processing portion 92.

For example, as shown below, subtraction may be performed in the R and B images and addition be performed in the G image. This processing enhances the lesion area with green.

$$R_D = R_N \times \left| 1 - \alpha \times \frac{R_F}{G_F} \right|$$

$$G_D = G_N \times \left| 1 + \alpha \times \frac{R_F}{G_F} \right|$$

$$B_D = B_N \times \left| 1 - \alpha \times \frac{R_F}{G_F} \right|$$

Alternatively, as shown below, subtraction may be performed in the R and G images and addition be performed in the B image. This processing enhances the lesion area with blue.

$$R_D = R_N \times \left| 1 - \alpha \times \frac{R_F}{G_F} \right|$$

$$G_D = G_N \times \left| 1 - \alpha \times \frac{R_F}{G_F} \right|$$

$$B_D = B_N \times \left| 1 + \alpha \times \frac{R_F}{G_F} \right|$$

Alternatively, as shown below, addition may be performed in the R and G images and subtraction be performed in the B image. This processing enhances the lesion area with yellow.

$$R_D = R_N \times \left| 1 + \alpha \times \frac{R_F}{G_F} \right|$$

$$G_D = G_N \times \left| 1 + \alpha \times \frac{R_F}{G_F} \right|$$

$$B_D = B_N \times \left| 1 - \alpha \times \frac{R_F}{G_F} \right|$$

Alternatively, as shown below, addition may be performed in the R and B images and subtraction be performed in the G image.
This processing enhances the lesion area with magenta.

$$R_D = R_N \times \left| 1 + \alpha \times \frac{R_F}{G_F} \right|$$

$$G_D = G_N \times \left| 1 - \alpha \times \frac{R_F}{G_F} \right|$$

$$B_D = B_N \times \left| 1 + \alpha \times \frac{R_F}{G_F} \right|$$

Alternatively, as shown below, subtraction may be performed in the R image and addition be performed in the B and G images. This processing enhances the lesion area with cyan.

$$R_D = R_N \times \left| 1 - \alpha \times \frac{R_F}{G_F} \right|$$

$$G_D = G_N \times \left| 1 + \alpha \times \frac{R_F}{G_F} \right|$$

$$B_D = B_N \times \left| 1 + \alpha \times \frac{R_F}{G_F} \right|$$

Alternatively, as shown below, subtraction may be performed in all of the R, B and G images. According to this processing, the displayed lesion area is darker than the normal area.

$$R_D = R_N \times \left| 1 - \alpha \times \frac{R_F}{G_F} \right|$$

$$G_D = G_N \times \left| 1 - \alpha \times \frac{R_F}{G_F} \right|$$

$$B_D = B_N \times \left| 1 - \alpha \times \frac{R_F}{G_F} \right|$$

Alternatively, as shown below, addition may be performed in all of the R, B and G images. According to this processing, the displayed lesion area is brighter than the normal area.

$$R_D = R_N \times \left| 1 + \alpha \times \frac{R_F}{G_F} \right|$$

$$G_D = G_N \times \left| 1 + \alpha \times \frac{R_F}{G_F} \right|$$

$$B_D = B_N \times \left| 1 + \alpha \times \frac{R_F}{G_F} \right|$$

As described above, the coefficient $\alpha$ is preferably used and is not essential. Depending on the foregoing device characteristics of the endoscope system 10, $R_F/G_F$ may take a value close to 1 in the lesion area and optionally a value close to 0 in the normal area without using the coefficient $\alpha$.

The processing of the normal images $R_N$, $G_N$ and $B_N$ includes the addition or subtraction of $R_F/G_F$ to or from 1 but the invention is not limited thereto. For example, $R_F/G_F$ may be directly multiplied by the normal images to calculate the images $R_D$, $G_D$ and $B_D$. Alternatively, $R_F/G_F$ may be directly added to or subtracted from the normal images to calculate the images $R_D$, $G_D$ and $B_D$.

In addition, in the above examples, all of the R, G and B images are processed with a correction factor including $R_F/G_F$ to calculate the images $R_D$, $G_D$ and $B_D$. However, the invention is not limited to this method.

For example, in the foregoing processing (operation as shown in any of the eight formulas), $R_F/G_F$ may be added ($1 + R_F/G_F$ be multiplied) in the R image, be subtracted ($1 - R_F/G_F$ be multiplied) in the G image and not be processed in the B image to calculate the images $R_D$, $G_D$ and $B_D$, respectively. Alternatively, $R_F/G_F$ may only be added in the color(s) that one wants to enhance.

As described above, in the case of the normal light observation, the normal images $R_N$, $G_N$ and $B_N$ read out by the read-out portion 90 are not processed in the fluorescence processing portion 92 and are supplied to the image correcting portion 94 as the images $R_D$, $G_D$ and $B_D$. In the case of the fluorescence observation, the normal images $R_N$, $G_N$ and $B_N$ read out by the read-out portion 90 are processed with $R_F/G_F$ in the fluorescence processing portion 92 and supplied to the image correcting portion 94 as the images $R_D$, Go and $B_D$.

The images $R_D$, $G_D$ and $B_D$ are subjected to predetermined processing such as color conversion or image structure enhancement in the image correcting portion 94 before being supplied to the display image generating section 80 as images for display (normal light observation images/fluorescence images).

The display image generating section 80 subjects the normal light observation images and/or fluorescence images supplied from the image generating section 76 to color space conversion, scaling and other necessary processing steps, or image allocation, incorporation of character information such as the name of a subject and other necessary processing steps to generate a display image having the normal light observation images and/or fluorescence images incorporated therein and this image is displayed on the monitor 18.

The endoscope system 10 of the invention can display only the normal light observation images or only the fluorescence images but the invention is not limited thereto. For example, the fluorescence images obtained by processing in the fluorescence processing portion 92 and the normal light observation images which are not processed in the fluorescence processing portion 92 may be displayed side by side (in parallel). The toggle display of the fluorescence images and the normal light observation images is also possible. In addition, the display modes including at least two of display of the fluorescence images, display of the normal light images, parallel display of the fluorescence images and the normal light images and toggle image display may be provided so that one of them can be selected.

An example of the operation of the endoscope system 10 is described below for the case of the fluorescence observation.

When the input device 20 issues an instruction for the start of imaging with the endoscope 12, the white light source 60 and the excitation light source 62 of the light source device 16 are alternately turned on at predetermined intervals. In the endoscope 12, the CCD sensor 48 and the high-sensitivity sensor 50 simultaneously start imaging.

The white light emitted from the white light source 60 is propagated through the optical fiber 60a and is supplied through the connection portion 16a to the connector 32 of the endoscope 12. The excitation light emitted from the excitation light source 62 is propagated through the optical fiber 62a and is supplied through the connection portion 16a to the connector 32 of the endoscope 12.

The white light and excitation light supplied to the connector 32 of the endoscope 12 is propagated through the optical fiber 58 to the scope portion 42, where the white light and excitation light exit from the distal end of the optical fiber 58 to be irradiated on the observation site of the living body.

The CCD sensor 48 captures the white light irradiated on the observation site to output R, G and B images of the observation site (reflected light from the living body). The high-sensitivity sensor 50 captures the excitation light irradiated on the observation site to output R and G images of the fluorescence from the living tissue of the observation site.

Output signals from both the sensors are supplied to the AFE board 64. The AFE board 64 subjects the output signals from the sensors to noise removal by correlated double sampling, amplification and A/D conversion to obtain digital image signals, which are then supplied to the DSP 72 of the processing device 14 (processor 14a).

The DSP 72 subjects the supplied images (image signals) to predetermined processing such as gamma correction and color correction and the processed images are stored in a predetermined portion of the storage section 74.

Once the image signals are stored in the storage section 74, the read-out portion 84 of the fluorescence image processing section 78 reads out the R fluorescence image $R_F$ and the G fluorescence image $G_F$ captured by the high-sensitivity sensor 50 and supplies them to the $R_F/G_F$ arithmetic portion 86.

The $R_F/G_F$ arithmetic portion 86 calculates $R_F/G_F$ and supplies it to the fluorescence processing portion 92 (R image processing portion 92r, G image processing portion 92g and B image processing portion 92b) of the image generating section 76.

On the other hand, the read-out portion 90 of the image generating section 76 reads out the R normal image $R_N$, G normal image $G_N$ and B normal image $B_N$ captured by the CCD sensor 48 and supplies them to the fluorescence processing portion 92.

In the fluorescence processing portion 92, the R image processing portion 92r, the G image processing portion 92g and the B image processing portion 92b process the normal image $R_N$, normal image $G_N$ and normal image $B_N$ by the following formulas using $R_F/G_F$ to obtain the images $R_D$, $G_D$ and $B_D$.

$$R_D = R_N \times \left| 1 + \alpha \times \frac{R_F}{G_F} \right|$$

$$G_D = G_N \times \left| 1 - \alpha \times \frac{R_F}{G_F} \right|$$

$$B_D = B_N \times \left| 1 - \alpha \times \frac{R_F}{G_F} \right|$$

The images $R_D$, $G_D$ and $B_D$ obtained in the fluorescence processing portion 92 are then subjected to predetermined image correction steps such as color conversion and image structure processing in the image correcting portion 94 and supplied to the display image generating section 80. The display image generating section 80 subjects the images $R_D$, $G_D$ and $B_D$ to predetermined processing steps and incorporates information such as the name of a patient to generate a display image. In addition, the display image generating section 80 causes the generated display image to be displayed on the monitor 18 as a fluorescence image.

As described above, the display image is an image in which the normal light observation images are included as the base and the lesion area captured in the fluorescence observation mode is enhanced with red, and therefore the normal area and lesion area are easily identified and the brightness is at the same level as that of the normal light observation images.

While the endoscope system of the invention has been described above in detail, the invention is by no means limited to the above embodiments, and various improvements and modifications may of course be made without departing from the spirit of the invention.

**Claims**

1. An endoscope system comprising:

   a light source device comprising a light source for fluorescence observation which emits excitation light exciting a fluorescence component in a living body to cause the fluorescence component to emit fluorescence and a light source for normal light observation which emits white light;
   an endoscope which receives said excitation light and said white light from said light source device, irradiates said excitation light and said white light into the living body and photoelectrically captures images in the living body; and
   processing means for processing normal light observation images captured in said endoscope by irradiation with said white light, using a G fluorescence signal and an R fluorescence signal obtained by capturing in said endoscope the fluorescence emitted from said fluorescence component in the living body irradiated with said excitation light.

2. The endoscope system according to claim 1, wherein said light source for fluorescence observation emits light at a wavelength of 370 to 470 nm as said excitation light.

3. The endoscope system according to claim 1 or 2, wherein said processing means uses a ratio of the R fluorescence signal to the G fluorescence signal obtained by dividing the R fluorescence signal by the G fluorescence signal to process said normal light observation images.

4. The endoscope system according to claim 3, wherein said processing means multiplies at least one of normal image signals including an R image signal, a G image signal and a B image signal by a correction factor including said ratio of the R fluorescence signal to the G fluorescence signal to process said normal light observation images.

5. The endoscope system according to claim 4, wherein said processing means multiplies at least one of said normal image signals including the R image signal, the G image signal and the B image signal by the correction factor obtained by adding said ratio of the R fluorescence signal to the G fluorescence signal to 1 to process said normal light observation images.

6. The endoscope system according to claim 5, wherein said processing means multiplies said ratio of the R fluorescence signal to the G fluorescence signal by a predetermined coefficient $\alpha$ and adds a resultant obtained by multiplication to 1 to generate said correction factor.

7. The endoscope system according to any one of claims 4 to 6, wherein said processing means multiplies at least one of said normal image signals including the R image signal, the G image signal and the B image signal by the correction factor obtained by subtracting said ratio of the R fluorescence signal to the G fluorescence signal from 1 to process said normal light observation images.

8. The endoscope system according to claim 7, wherein said processing means multiplies said ratio of the R fluorescence signal to the G fluorescence signal by a predetermined coefficient $\alpha$ and subtracts a resultant obtained by multiplication from 1 to generate said correction factor.

9. The endoscope system according to any one of claims 1 to 8, wherein said endoscope comprises:

   a first solid-state image sensor for capturing the normal light observation images by irradiation with said white light; and
   a second solid-state image sensor for capturing the fluorescence emitted from said fluorescence component in the living body by irradiation with said excitation light.

10. The endoscope system according to claim 9, wherein said endoscope uses one imaging lens to form the images in said first and second solid-state image sensors.

11. The endoscope system according to claim 9 or 10, wherein said second solid-state image sensor captures the fluorescence emitted from said fluorescence component in the living body via a filter through which said excitation light does not pass but said fluorescence emitted from said fluorescence component in the living body passes.

# FIG.1

FIG.2

FIG.3

FLUORESCENCE PROCESSING

FIG.4

**Europäisches Patentamt**
**European Patent Office**
**Office européen des brevets**

# EUROPEAN SEARCH REPORT

**Application Number**

EP 11 19 5902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/046778 A1 (ISHIHARA YASUSHIGE [JP] ET AL) 1 March 2007 (2007-03-01) | 1-4,9-11 | INV. A61B1/07 A61B1/06 A61B1/04 A61B1/00 |
| A | * paragraphs [0018] - [0019], [0207] - [0208], [0214] - [0215], [0224], [0237] - [0245] * <br> * figure 27 * | 5-8 | |
| A | US 5 647 368 A (ZENG HAISHAN [CA] ET AL) 15 July 1997 (1997-07-15) <br> * the whole document * | 1-11 | |
| A | US 2003/135092 A1 (CLINE RICHARD W [CA] ET AL) 17 July 2003 (2003-07-17) <br> * the whole document * | 1-11 | |

**TECHNICAL FIELDS SEARCHED (IPC)**

A61B

The present search report has been drawn up for all claims

| Place of search | Date of completion of the search | Examiner |
|---|---|---|
| The Hague | 7 May 2012 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 19 5902

This annex lists the patent family members relating to the patent documents cited in  the above-mentioned European search report.
The members are as contained in the European Patent Office EDP file on
The European Patent Office is in no way liable for these particulars which are merely given for the purpose of information.

07-05-2012

| Patent document cited in search report | | Publication date | Patent family member(s) | | Publication date |
|---|---|---|---|---|---|
| US 2007046778 | A1 | 01-03-2007 | EP | 1759628 A1 | 07-03-2007 |
| | | | JP | 2007090044 A | 12-04-2007 |
| | | | US | 2007046778 A1 | 01-03-2007 |
| US 5647368 | A | 15-07-1997 | DE | 69725361 D1 | 13-11-2003 |
| | | | DE | 69725361 T2 | 15-07-2004 |
| | | | EP | 0792618 A1 | 03-09-1997 |
| | | | JP | 3022377 B2 | 21-03-2000 |
| | | | JP | 9327433 A | 22-12-1997 |
| | | | US | 5647368 A | 15-07-1997 |
| US 2003135092 | A1 | 17-07-2003 | EP | 1476071 A2 | 17-11-2004 |
| | | | EP | 1477103 A1 | 17-11-2004 |
| | | | EP | 1520508 A1 | 06-04-2005 |
| | | | JP | 4444657 B2 | 31-03-2010 |
| | | | JP | 2005514147 A | 19-05-2005 |
| | | | US | 2003135092 A1 | 17-07-2003 |
| | | | US | 2005143627 A1 | 30-06-2005 |
| | | | US | 2005154319 A1 | 14-07-2005 |
| | | | WO | 03059159 A2 | 24-07-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3810337 B
- JP 2006043289 A
- JP 2009056248 A